# EUROPÄISCHE PATENTSCHRIFT

(11) **EP 0 543 827 B1**
(45) Veröffentlichungstag und Bekanntmachung des Hinweises auf die Patenterteilung: **18.05.1994**
(21) Anmeldenummer: 91912907.2
(22) Anmeldetag: 19.07.1991
(51) Int. Cl.: A61B 5/021

(54) **BLUTDRUCKMESSGERÄT**
BLOOD PRESSURE MEASURING DEVICE
APPAREIL DE MESURE DE LA TENSION ARTERIELLE

(30) Priorität: 18.08.1990 DE 4026242
(43) Veröffentlichungstag der Anmeldung: 02.06.1993
(73) Patentinhaber: Speidel + Keller GmbH + Co. KG, D-72417 Jungingen (DE)
(72) Erfinder: SPEIDEL, Blasius, D-7455 Jungingen (DE)
(74) Vertreter: Kastner, Hermann, Dipl.-Ing. Patentanwalt
(86) Internationale Anmeldenummer: EP9101358
(87) Internationale Veröffentlichungsnummer: WO9203087

(56) Entgegenhaltungen:
- EP-A- 0 008 351
- FR-A- 1 535 709
- FR-A- 2 275 182

## Beschreibung

Bei den meisten bekannten Blutdruckmeßgeräten ist das Druckmeßwerk in einem üblicherweise becherförmigen Gehäuse untergebracht und darin an einem der Teile des Gehäuses befestigt. Dieses Gehäuse nimmt auch die Meßskala auf, durch die sich die Meßwerkswelle hindurcherstreckt. Oberhalb der Maßskala ist der Meßzeiger auf der Meßwerkswelle angebracht. Die Meßskala und der Meßzeiger werden durch eine durchsichtige Abdeckscheibe abgedeckt, die entweder unmittelbar am oberen Rand des Gehäuses eingesetzt ist oder die in einem gesonderten Haltering eingesetzt ist, der seinerseits mit dem Rand des Gehäuses verbunden ist (EP-A-0 008 351 A2). In beiden Fällen schließt die Abdeckscheibe den Innenraum des Gehäuses mit den darin untergebrachten Teilen nach außen ab.

Wenn ein solches Blutdruckmeßgerät auf einen harten Untergrund herabfällt, besteht die Gefahr, daß das Gehäuse beschädigt wird, insbesondere verformt wird. Im Bereich des Bodens und des Überganges des Bodens in die Umfangswand hat das Gehäuse von Natur aus eine verhältnismäßig hohe Formfestigkeit, so daß in diesem Bereich die durch das Herabfallen verursachten Verformungen im allgemeinen verhältnismäßig gering bleiben. Anders verhält es sich, wenn das Gehäuse im Bereich seines oberen Randes aufschlägt, wo die Meßskala und die Abdeckscheibe eingesetzt sind. Dabei besteht zugleich die Gefahr, daß neben dem Gehäuse selbst auch die Meßskala und die Abdeckscheibe beschädigt werden. Die Meßskala kann verbogen oder verwölbt werden. Diese Verformung der Meßskala kann so weit gehen, daß der Meßzeiger sich nicht mehr ungehindert über die Meßskala hinwegbewegen kann.

Bei einer Verformung des Gehäuses kann die Meßskala auch aus ihrer Halterung herausspringen. Das gleiche gilt auch für die Abdeckscheibe. Bei einem besonders starken Stoß kann auch die als Spiralfeder ausgebildete Rückholfelder des Meßwerks beschädigt werden, wenn z.B. eine oder mehrere ihrer Windungen übereinanderschlagen und sich dann verhaken. Bei allen diesen Beschädigungen oder gar Zerstörungen einzelner Teile wird in der Regel das gesamte Blutdruckmeßgerät unbrauchbar.

Der Erfindung liegt die Aufgabe zugrunde, ein Blutdruckmeßgerät so auszubilden, daß die von einem Fall ausgehenden Beschädigungen oder Zerstörungen zumindest vermindert sind, wenn nicht gar ganz vermieden werden.

Dadurch, daß das Primärgehäuse von einem Schutzgehäuse umgeben ist, kann beim Herabfallen des Blutdruckmeßgerätes auf einen harten Untergrund die Umfangswand des innengelegenen Primär gehäuses nicht aufschlagen. Sie kann dadurch nicht verformt werden. Dadurch ist die Gefahr nahezu ausgeschlossen, daß das im Primärgehäuse untergebrachte Meßwerk und seine Teile beschädigt werden. Insbesondere wird vermieden, daß das Primärgehäuse im Bereich seines oberen Randes eingedrückt wird, wo die Meßskala und die Abdeckscheibe befestigt sind. Daher können auch diese Teile praktisch nicht mehr beschädigt werden. Dadurch, daß der Boden des Schutzgehäuses zumindest annähernd auf gleicher Höhe mit dem Boden des Primärgehäuses an der Außenseite dessen Umfangswand einstückig anschließt, wird die Formfestigkeit des Gesamtgehäuses in diesem Bereich erheblich vergrößert. Allein dadurch schon sind Verformungen in diesem Bereich zumindest am Primärgehäuse völlig ausgeschlossen und die Gefahr von Verformungen auch am Schutzgehäuse sehr stark vermindert.

Bei einer Ausgestaltung des Blutdruckmeßgerätes nach Anspruch 2 wird ein einfacher Anschluß des Druckmeßwerkes an die an der Außenseite des Bodens des Gehäuses angeordneten sonstigen Teile des Blutdruckmeßgerätes erleichtert. Bei einer alternativen Ausgestaltung des Blutdruckmeßgerätes nach Anspruch 3 wird das gleiche für einen Anschluß im Bereich der Seitenwand des Gesamtgehäuses erreicht, wenn dort die weiteren Teile des Blutdruckmeßgerätes angeschlossen sind.

Bei einer Ausgestaltung des Blutdruckmeßgerätes nach Anpruch 4 wird durch den am oberen Rand des Schutzgehäuses angeordneten Schutzring der Zwischenraum zwischen der Umfangswand des Primärgehäuses und der Umfangswand des Schutzgehäuses nach außen hin abgedeckt. Dadurch wird ein Eindringen von Staub und Schmutz in diesen Zwischenraum zumindest stark vermindert, wenn nicht gar ganz ausgeschlossen. Außerdem wird dadurch, daß der Schutzring sich einwärt bis mindestens zum Rand des Primärgehäuses, wenn nicht gar etwas darüber hinaus erstreckt, der obere Rand des Primärgehäuses mit der dort eingesetzten Abdeckscheibe und der darunter eingesetzten Meßskala auch von der Stirnseite des Blutdruckmeßgerätes her geschützt.

Bei einer Weiterbildung des Blutdruckmeßgerätes nach Anspruch 5 wird die Gefahr von Beschädigungen des Schutzringes verringert, weil durch dessen sich verjüngende Außenseite keine scharfe Kante mehr vorhanden ist, die beim Aufschlagen auf einen harten Untergrund eher einer Beschädigung ausgesetzt wäre. Durch diese Formgebung des Schutzringes wird die Aufschlagfläche vergrößert und damit die Flächenbelastung an der Aufschlagstelle vermindert. Außerdem wird dadurch ein leichteres Abrollen des Gehäuses auf dem Untergrund erleichtert, was ebenfalls zur Verminderung von Belastungsspitzen beiträgt.

Durch eine Ausgestaltung des Blutdruckmeßgerätes nach Anspruch 6 wird die Montage des Schutzringes vereinfacht und erleichtert.

Bei einer Ausgestaltung des Blutdruckmeßgerätes nach Anspruch 7 wird durch den elastischen Zwischenring zwischen dem Schutzring und dem Schutzgehäuse ein Teil der Stoßenergie in dem Falle absorbiert, daß das Gehäuse des Blutdruckmeßgerätes mit dem Schutzring voran auf eine harte Unterlage aufschlägt. Auch dadurch wird die Gefahr von Beschädigungen der Teile des Blutdruckmeßgerätes erheblich verringert.

Im folgenden wird die Erfindung anhand zweier in der Zeichnung dargestellter Ausführungsbeispiele näher erläutert. Es zeigen:
- Fig. 1: eine teilweise geschnitten dargestellte Ansicht eines Gehäuses des Blutdruckmeßgerätes, bei der die meisten Teile des Meßwerkes nicht dargestellt sind;
- Fig. 2: eine teilweise geschnitten dargestellte Ansicht des Gehäuses eines zweiten Ausführungsbeispieles des Blutdruckmeßgerätes;
- Fig. 3: eine Ansicht des Gehäuses nach Fig. 2 in einer um 90° um seine Achse gedrehten Darstellung.

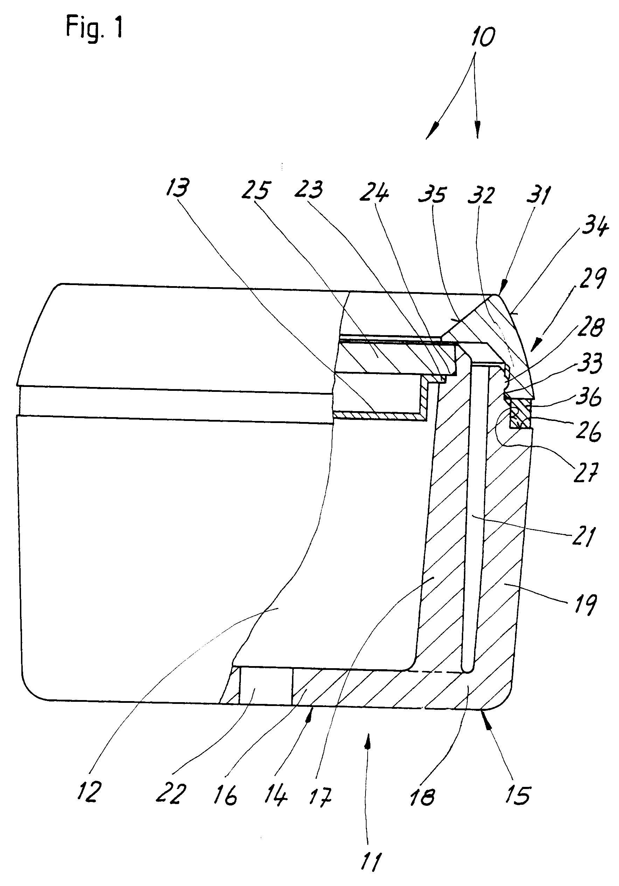

Das aus Fig. 1 ausschnittweise ersichtliche Blutdruckmeßgerät 10 weist ein zumindest näherungsweise becherförmiges Gehäuse 11 auf. In dessen Innenraum 12 ist das Druckmeßwerk herkömmlicher Bauart untergebracht, von dem in Fig. 1 nur die Meßskala 13 dargestellt ist.

Das Gehäuse 11 ist eine Art Doppelgehäuse mit einem inneren oder primären Gehäuse, das im folgenden als Primärgehäuse 14 bezeichnet wird, und mit einem äußeren oder sekundären Gehäuse, das im folgenden seiner besonderen Funktion wegen als Schutzgehäuse 15 bezeichnet wird. Das Gehäuse 11 mit seinen beiden Teilgehäusen 14 und 15 ist als Kunststoffformteil in einem Stück hergestellt.

Das Primärgehäuse 14 ist becherförmig und weist einen Boden 16 und eine Umfangswand 17 auf. Das Schutzgehäuse 15 hat zumindest annähernd die gleiche Form und weist ebenfalls einen Boden 18 und eine Umfangswand 19 auf. Der Boden 18 des Schutzgehäuses 15 schließt auf gleicher Höhe mit dem Boden 16 des Primärgehäuses 14 an der Außenseite dessen Umfangswand 17 an. Dadurch verringert sich der Boden 18 auf eine Kreisringfläche, deren radiale Erstreckung nur etwa so groß ist, wie die lichte radiale Erstreckung des Zwischenraumes 21 zwischen der Umfangswand 17 des Primärgehäuses und der Umfangswand 19 des Schutzgehäuses 15.

Das Primärgehäuse 14 weist in der Kreisringmitte seines Bodens 16 ein kreiszylindrisches Durchgangsloch 22 auf, durch das die druckluftführenden Teile des Blutdruckmeßgerätes 10 mit dem im Innenraum 12 untergebrachten Druckmeßwerk in Verbindung steht.

Am oberen Rand der Umfangswand 17 des Primärgehäuses 14 sind auf der Innenseite der Umfangswand 17 zwei im Durchmesser gegeneinander abgestufte Absätze 23 und 24 vorhanden. Auf dem unteren inneren Absatz 23 liegt der Rand der Meßmembran 13 auf. Auf dem oberen äußeren Absatz 24 liegt der Rand einer durchsichtigen Abdeckscheibe 25 auf. Die Umfangsfläche der Abdeckscheibe 25 und der an den Absatz 24 anschließende Flächenabschnitt der Innenseite der Umfangswand 17 sind als Kegelstumpfmantelflächen mit einem sehr kleinen Kegelspitzenwinkel ausgebildet. Dadurch entsteht eine Art Schnappverschluß, der die Abdeckscheibe 25 am oberen Rand des Primärgehäuses 14 festhält, wenn sie dort eingesetzt worden ist. Die Abdeckscheibe 25 hält dann zugleich auch die Meßskala 13 an ihrem Platz fest.

An der Umfangswand 19 des Schutzgehäuses 15 ist im Bereich des oberen Randes auf der Außenseite ein Absatz 26 mit einer ebenen Kreisringfläche vorhanden. Er wird innen von einer Kreiszylinderfläche 27 begrenzt. In dem daran anschließenden Längenabschnitt der Umfangswand 19, dessen Außenseite zumindest näherungsweise Kreiszylinderform hat, ist ein radial nach außen vorstehende Ringwulst 28 angeformt. Dieser bildet als Wandvorsprung den einen Teil eines Schnappverschlusses 29, durch den ein Schutzring 31 festgehalten wird, der auf die Umfangswand 19 des Schutzgehäuses 15 von oben her aufgesetzt ist. Am Schutzring 31 ist auf der Innenseite einer Umfangswand 32 ein nach innen vorstehender Ringwulst 33 angeformt, der den Ringwulst 28 an der Umfangswand 19 hintergreift und als Rücksprung des Schnappverschlusses 29 wirkt.

Der Schutzring 31 hat einen dachförmigen Querschnitt mit unterschiedlicher Dachflächenhöhe. Die außengelegene Dachfläche oder kurz Außenseite 34 des Schutzringes 31, hat zumindest näherungsweise die Form eines Kugelabschnittes. Die innengelegene Dachflächenseite oder kurz Innenseite 35 des Schutzringes 31 hat die Form einer Kegelstumpfmantelfläche.

Wie aus Fig. 1 zu ersehen ist, erstreckt sich der Schutzring 31 von der Außenseite der Umfangswand 19 des Schutzgehäuses 15 einwärts bis über die Umfangswand 17 des Primärgehäuses 14 hinweg, so daß er von der am Rand der Umfangswand 17 eingesetzte Abdeckscheibe 25 in radialer Richtung um eine schmale Kreisringfläche überdeckt. In axialer Richtung liegt der Rand des Schutzringes 31 nicht an der Abdeckscheibe 25 an, sondern hält einen kleinen Sicherheitsabstand zu ihr ein, damit der Schutzring 31 bei einer möglichen elastischen Verformung nicht sofort an der Abdeckscheibe 25 anschlägt.

Damit der Schutzring 31 seine Funktion optimal erfüllen kann, ist er zweckmäßigerweise aus einem Kunststoff hergestellt, der eine höhere Schlag- und Bruchfestigkeit als der Werkstoff des Gehäuses 11 hat.

Der Schnappverschluß 29 ist so ausgebildet, daß der Schutzring 31 um ein kleines Maß in axialer Richtung gegenüber dem Schutzgehäuse 15 verschoben werden kann, wenn er am Rand des Schutzgehäuses 15 eingeschnappt ist. Zwischen dem Absatz 26 des Schutzgehäuses 15 und der nach unten gerichteten Stirnfläche der Umfangswand 32 des Schutzringes 31 ist ein zumindest in axialer Richtung elastischer Zwischenring 36 eingefügt. Dieser dämpft Stöße ab, die unter Umständen auf den Schutzring 31 von oben her auftreffen.

Anstelle des Zwischenringes 16 aus einem Elastomer kann auch ein Zwischenring aus einem weniger elastischen bis starren Werkstoff eingesetzt werden, der aufgrund seines Werkstoffes, beispielsweise als Metallring, oder aufgrund einer gesonderten Farbgebung einen bestimmten Farbkontrast zur Farbe des Schutzgehäuses 15 und/oder zur Farbe des Schutzringes 31 abgibt.

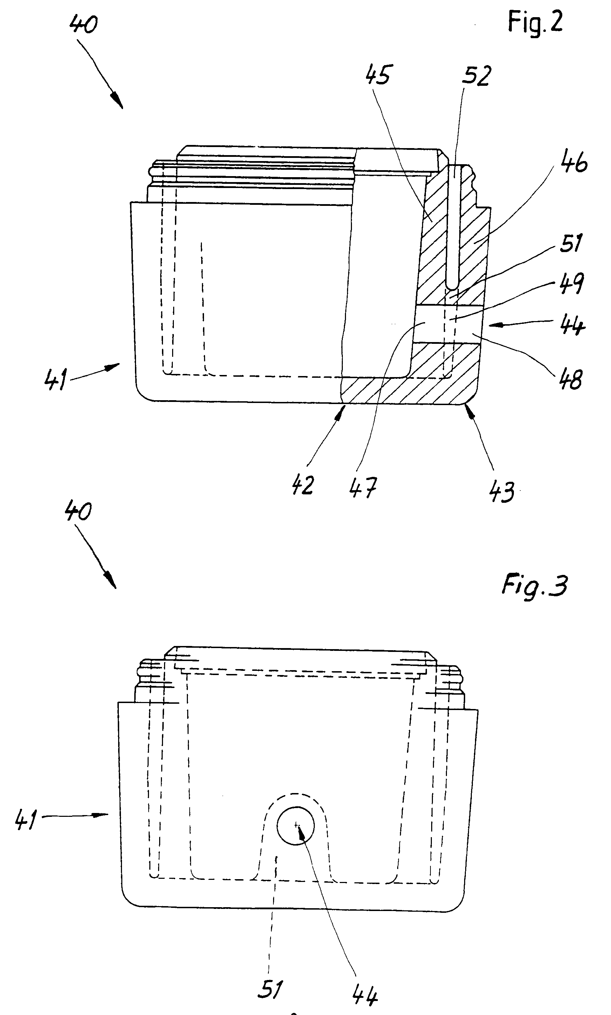

Bei dem aus Fig. 2 und 3 ausschnittweise ersichtlichen Blutdruckmeßgerät 40 ist lediglich das Gehäuse 41 in gewissen Teilen gegenüber dem Gehäuse 11 abgewandelt. Die übrigen Teile des Blutdruckmeßgerätes 40, die sämtlich nicht dargestellt sind, sind als gleich oder zumindest ähnlich den entsprechenden Teilen des Blutdruckmeßgerätes 10 zu betrachten.

Die Außen- und Innenmaße des Gehäuses 41 sind praktisch gleich denen des Gehäuses 11. Es weist ebenso zwei Teilgehäuse auf, nämlich das Primärgehäuse 42 und das Schutzgehäuse 43.

Das im Inneren des Gehäuses 41 untergebrachte Druckmeßwerk ist hier nicht über ein Durchgangsloch im Boden des Gehäuses mit den übrigen druckluftführenden Teilen des Blutdruckmeßgerätes 40 verbunden sondern über eine Anschlußleitung 44, die im Bereich der Umfangswand 45 des Primärgehäuses 42 und der Umfangswand 46 des Schutzgehäuses 43 angeordnet ist. Diese Anschlußleitung 44 wird gebildet durch ein Durchgangsloch 47 in der Umfangswand 45, durch ein Durchgangsloch 48 in der Umfangswand 46 und durch ein Durchgangsloch 49 in einer Materialbrücke 51. Diese Materialbrücke 51 füllt im Bereich der Anschlußleitung 47 ringsum den Zwischenraum 52 zwischen der Umfangswand 45 des Primärgehäuses 42 und der Umfangswand 46 des Schutzgehäuses 43 aus und schließt dabei die Anschlußleitung 44 nach außen hin ab.

## Patentansprüche

1. Blutdruckmeßgerät mit den Merkmalen:
- es ist ein primäres becherförmiges Gehäuse (14) mit einem Boden (16) und einer Umfangswand (17) vorhanden,
- im Innenraum (12) des Gehäuses (14) ist ein Druckmeßwerk mit Meßwerkswelle angeordnet, das an einem Gehäuseteil befestigt ist,
- im Bereich des oberen Randes des Gehäuses (14) ist eine Meßskala (13) angeordnet, durch die die Meßwerkswelle des Druckmeßwerkes sich hindurcherstreckt,
- oberhalb der Meßskala (13) ist ein Meßzeiger mit der Meßwerkswelle drehfest verbunden,
- am oberen Rand des Gehäuses (14) ist eine durchsichtige Abdeckscheibe (25) angeordnet, die mit dem Rand des Gehäuses (14) abnehmbar verbunden ist,
**gekennzeichnet** durch die Merkmale:
- neben dem primären Gehäuse (14) (Primärgehäuse) mit dem Meßwerk ist ein sekundäres Gehäuse (15) (Schutzgehäuse) vorhanden,
- die Umfangswand (19) des Schutzgehäuses (15) umgibt die Umfangswand (17) des Primärgehäuses (14) in einem bestimmten lichten radialen Abstand,
- der Boden (18) des Schutzgehäuses (15) schließt zumindest annähernd auf gleicher Höhe mit dem Boden (16) des Primärgehäuses (14) an der Außenseite dessen Umfangswand (17) einstückig an.

2. Blutdruckmeßgerät nach Anspruch 1,
**gekennzeichnet** durch das Merkmal:
- für den Anschluß der Druckluftleitung von der Meßmanschette zum Druckmeßwerk ist im Boden (16) des Primärgehäuses (14) ein vorzugsweise kreiszylindrisches Durchgangsloch (22) vorhanden.

3. Blutdruckmeßgerät nach Anspruch 1,
**gekennzeichnet** durch die Merkmale:
- für den Anschluß der Druckluftleitung von der Meßmanschette zum Druckmeßwerk ist in der Umfangswand (45) des Primärgehäuses (42) und damit fluchtend in der Umfangswand (46) des Schutzgehäuses (43) je ein Durchgangsloch (47; 48) vorhanden,
- im Zwischenraum (52) zwischen der Umfangswand (45) des Primärgehäuses (42) und der Umfangswand (46) des Schutzgehäuses (43) ist im Bereich der beiden Durchgangslöcher (47; 48) eine Materialbrücke (51) vorhanden, vorzugsweise einstückig angeformt, die den zwischen den beiden Durchgangslöchern (47; 48) gelegen Längenabschnitt (49) der Druckluftleitung (44) außen dicht umschließt.

4. Blutdruckmeßgerät nach einem der Ansprüche 1 bis 3,
**gekennzeichnet** durch die Merkmale:
- am oberen Rand der Umfangswand (19) des Schutzgehäuse (15) ist ein Schutzring (31) angeordnet, der sich von der Außenseite der Umfangswand (19) des Schutzgehäuses (15) aus einwärts bis mindestens zum Rand des Primärgehäuses (14) hin, höchstens jedoch bis zum Schriftfeld der Meßskala (13) hin, erstreckt,
- vorzugsweise ist der Schutzring (31) aus einem Werkstoff hergestellt, der eine größere Schlag- und Bruchfestigkeit als der Werkstoff des Schutzgehäuses (15) hat.

5. Blutdruckmeßgerät nach Anspruch 4,
**gekennzeichnet** durch die Merkmale:
- die Außenseite (34) des Schutzringes (31) ist von dem dem Schutzgehäuse (15) zugekehrten Rand aus verjüngt,
- vorzugsweise ist die Außenseite des Schutzringes (31) als Kegelstumpfmantelfläche oder als Kugelabschnittfläche oder als Mischform beider Grundformen ausgebildet.

6. Blutdruckmeßgerät nach Anspruch 4 oder 5,
**gekennzeichnet** durch die Merkmale:
- der Schutzring (31) ist mittels eines Schnappverschlusses (29) mit zumindest zum Teil umlaufenden Vorsprüngen (28) und Rücksprüngen (33) mit dem Schutzgehäuse (15) verbunden.

7. Blutdruckmeßgerät nach Anspruch 6,
**gekennzeichnet** durch die Merkmale:
- der Schnappverschluß (29) ist selbst in axialer Richtung nachgiebig ausgebildet oder mit einer axialen Bewegungsmöglichkeit zwischen dem Schutzring (31) und dem Schutzgehäuse (15) versehen,
- zwischen zwei einander zugekehrten Ringflächen des Schutzringes (31) und des Schutzgehäuses (15) ist ein zumindest in axialer Richtung elastisch nachgiebiger Zwischenring (36) angeordnet.

## Claims

1. A blood pressure measuring apparatus with the features:
- a primary, cup-shaped housing (14) having a base (16) and a circumferential wall (17) is present,
- a pressure measurement mechanism with a measurement mechanism shaft is arranged in the interior (12) of the housing (14) and is secured to a housing part,
- in the region of the upper edge of the housing (14) there is arranged a measuring scale (13) through which there extends the measurement mechanism shaft of the pressure measurement mechanism,
- above the measuring scale (13), a measurement pointer is connected in a rotationally rigid manner to the measurement mechanism shaft,
- at the upper edge of the housing (14) there is arranged a transparent cover disc (25) which is detachably connected to the edge of the housing (14),
characterised by the features:
- in addition to the primary housing (14) with the measurement mechanism, there is a secondary housing (15) (protective housing),
- the circumferential wall (19) of the protective housing (15) surrounds the circumferential wall (17) of the primary housing (14) at a given, clear, radial distance therefrom,
- at least substantially level with the base (16) of the primary housing (14), the base (18) of the protective housing (15) is integrally connected to the exterior of the circumferential wall (17) of the primary housing (14).

2. A blood pressure measuring apparatus in accordance with Claim 1,
characterised by the feature:
- a preferably circular-cylindrical through bore (22) is present in the base (16) of the primary housing (14) for the purpose of connecting the compressed-air line from the measuring cuff to the pressure measurement mechanism.

3. A blood pressure measuring apparatus in accordance with Claim 1,
characterised by the features:
- for connecting the compressed-air line from the measuring cuff to the pressure measurement mechanism, a through bore (47; 48) is present in the circumferential wall (45) of the primary housing (42) and in alignment therewith in the circumferential wall (46) of the protective housing (43) respectively,
- in the interspace (52) between the circumferential wali (45) of the primary housing (42) and the circumferential wall (46) of the protective housing (43) there is a material bridge (51) present in the region of the two through bores (47; 48), preferably integrally moulded, and it surrounds the longitudinal portion (49) - located between the two through bores (47; 48) - of the compressed-air line (44) in a manner so as to render it impervious to the exterior.

4. A blood pressure measuring apparatus in accordance with any one of Claims 1 to 3,
characterised by the features:
- at the upper edge of the circumferential wall (19) of the protective housing (15) there is ananged a protective ring (31) which extends inwards from the exterior of the circumferential wall (19) of the protective housing (15) at least as far as the edge of the primary housing (14), at most however as far as the graphic region of the measuring scale (13),
- the protective ring (31) is preferably manufactured from a material which has greater impact resistance and resistance to breaking than the material of the protective housing (15).

5. A blood pressure measuring apparatus in accordance with Claim 4,
characterised by the features:
- the exterior (34) of the protective ring (31) tapers from the edge facing the protective housing (15),
- the exterior of the protective ring (31) is preferably in the form of a truncated cone surface or a surface of a spherical segment or a combination of both basic shapes.

6. A blood pressure measuring apparatus in accordance with Claim 4 or 5,
characterised by the features:
- the protective ring (31) is connected to the protective housing (15) by means of a snap-fit fastening (29) having at least partially encircling projections (28) and shoulders (33).

7. A blood pressure measuring apparatus in accordance with Claim 6,
characterised by the features:
- the snap-fit fastening (29) is itself designed to yield in the axial direction or is provided with the possibility for axial motion between the protective ring (31) and the protective housing (15),
- between two facing annular surfaces of the protective ring (31) and of the protective housing (15) there is arranged an intermediate ring (36) which is resiliently yielding in the axial direction at least.

## Revendications

1. Appareil de mesure de la tension artérielle avec les particularités suivantes:
- il y a un boîtier primaire en forme de cuvette (14) avec un fond (16) et une paroi périphérique (17),
- dans l'espace intérieur (12) du boîtier (14) on dispose un système de mesure avec un axe, qui est fixé sur une pièce du boîtier,
- dans la zone du bord supérieur du carter (14) est disposée une graduation (13), à travers laquelle passe l'axe du système de mesure de la tension artérielle,
- au-dessus de la graduation (13) une aiguille de mesure est reliée à l'axe du système de mesure,
- sur le bord supérieur du boîtier (14) on dispose un disque de recouvrement transparent (25) qui est relié au bord du boîtier (14) de façon amovible, caractérisé en ce qu'en plus du boîtier primaire (14) avec le système de mesure il y a un boîtier secondaire (15),
- la paroi périphérique (19) du boîtier de protection (15) entoure la paroi périphérique (17) du boîtier primaire (14) à une distance radiale libre déterminée,
- le fond (18) du boîtier de protection (15) se raccorde d'une seule pièce au moins approximativement à la même hauteur au fond (16) du boîtier primaire (14) sur le côté extérieur de sa paroi périphérique (17).

2. Appareil de mesure de la tension artérielle selon la revendication 1, caractérisé en ce que :
- pour le raccordement de la conduite d'air sous pression allant de la manchette de mesure au système de mesure de la pression il y dans le fond (16) du boîtier primaire (14) un trou de passage (22) ayant de préférence une forme cylindrique de section circulaire.

3. Appareil de mesure de la tension artérielle selon la revendication 1, caractérisé en ce que:
- pour le raccordement de la conduite d'air sous pression allant de la manchette de mesure au système de mesure de la pression il y a dans la paroi périphérique (45) du boîtier primaire (42) et venant de cette façon à fleur dans la paroi périphérique (46) du boîtier de protection (43) respectivement un trou de passage (47, 48),
- dans l'espace intermédiaire (52) entre la paroi périphérique (45) du boîtier primaire (42) et la paroi périphérique (46) du boîtier de protection (43) il y a dans la zone des deux trous de passage (47, 48) un pont de matière (51), de préférence formé par moulage d'une seule pièce, qui entoure de façon étanche extérieurement la section longitudinale (49) de la conduite d'air sous pression (44), mise entre les deux trous de passage (47, 48).

4. Appareil de mesure de la tension artérielle selon l'une des revendications 1 à 3, caractérisé en ce que:
- sur le bord supérieur de la paroi périphérique (19) du boîtier de protection (15) est disposé une bague de protection (31) qui s'étend depuis le côté extérieur de la paroi périphérique (19) du boîtier de protection (15) vers l'intérieur jusqu'au bord au moins, du boîtier primaire (14), mais toutefois au plus jusqu'à la zone d'inscription de la graduation (13),
- de préférence la bague de protection (31) est fabriquée en une matière, qui a une résistance au choc et à la rupture plus grande que la matière du boîtier de protection (15).

5. Appareil de mesure de la tension artérielle selon la revendication 4, caractérisé en ce que:
- le côté extérieur (34) de la bague de protection (31) va en se rétrécissant à partir du bord tourné vers le boîtier de protection (15),
- de préférence le côté extérieur de la bague de protection (31) est constituée avec une surface en forme de tronc de cône ou de section de sphère ou avec une forme mixte à partir des deux formes de base.

6. Appareil de mesure de la tension artérielle selon la revendication 4 ou 5, caractérisé en ce que:
- la bague de protection (31) est reliée par une fermeture à déclic (29) au boîtier de protection (15) par des saillies (28) et des retraits (33) qui font le tour au moins en partie.

7. Appareil de mesure de la tension artérielle selon la revendication 6, caractérisé en ce que:
- la fermeture à déclic (29) est constituée elle-même de façon flexible dans le sens axial ou est pourvue d'une possibilité de mouvement axiale entre la bague de protection (31) et le boîtier de protection (15),
- entre deux faces annulaires, tournées l'une vers l'autre, de la bague de protection (31) et du boîtier de protection (15) on dispose une bague intermédiaire (36) flexible élastiquement au moins dans le sens axial.
